# EUROPÄISCHE PATENTSCHRIFT

(11) **EP 0 779 836 B1**
(45) Veröffentlichungstag und Bekanntmachung des Hinweises auf die Patenterteilung: **10.03.1999**
(21) Anmeldenummer: 95931945.0
(22) Anmeldetag: 31.08.1995
(51) Int. Cl.: B01F 17/00, C11D 1/83

(54) **MILDE DETERGENSGEMISCHE**
MILD DETERGENT MIXTURES
MELANGES DE DETERGENTS DOUX

(30) Priorität: 09.09.1994 DE 4432130
(43) Veröffentlichungstag der Anmeldung: 25.06.1997
(73) Patentinhaber: Henkel Kommanditgesellschaft auf Aktien, 40191 Düsseldorf (DE)
(72) Erfinder: FABRY, Bernd, D-41352 Korschenbroich (DE); BEHLER, Ansgar, D-46240 Bottrop (DE)
(86) Internationale Anmeldenummer: EP9503413
(87) Internationale Veröffentlichungsnummer: WO9607473

(56) Entgegenhaltungen:
- EP-A- 0 117 135
- WO-A-92/09570
- WO-A-93/20171
- WO-A-95/23582
- DE-A- 1 467 564
- DE-A- 4 229 442
- DE-A- 4 300 325
- FR-A- 1 411 088
- GB-A- 824 654
- N.B.Desai: "Neue Saccharoseester und deren Verwendung in der Kosmetik, Parfum und Kosmetik, 64.Jahrgang, Nr.9, 1991, S.463-469

## Beschreibung

### Gebiet der Erfindung

Die Erfindung betrifft Detergensgemische mit verbesserter hautkosmetischer Verträglichkeit, enthaltend Monoglycerid(ether)sulfate und ausgewählte Zuckertenside sowie die Verwendung der Gemische zur Herstellung von oberflächenaktiven Mitteln.

### Stand der Technik

Monoglyceridsulfate stellen formal Anlagerungsprodukte von Schwefeltrioxid an die primäre Hydroxylgruppe eines Glycerinmonofettsäureesters dar. Technisch gesehen handelt es sich jedoch um komplexe Aniontensidgemische, die üblicherweise durch gleichzeitige Umesterung und Sulfatierung von Gemischen aus Trigylceriden und Glycerin sowie nachfolgender Neutralisation erhalten werden.

Monoglyceridsulfate zeichnen sich durch zufriedenstellende anwendungstechnische Eigenschaften und gute dermatologische Verträglichkeit aus. Übersichten zu Herstellung und Eigenschaften von Monoglyceridsulfaten sind beispielsweise von A.K.Biswas et al. in **J.Am.Oil.Chem.Soc. 37, 171 (1960),** R.Chamanial et al. in **J.Oil.Technol.Ass.Ind**. **41 (1972)** und J.K.Jain in **Indian J.Pharm.Sci. 41, 181 (1979)** erschienen.

Für eine Reihe von Anwendungen ist das Schaumvermögen, insbesondere bei Härtebelastung, sowie die hautkosmetische Verträglichkeit der Monoglyceridsulfate sowie der dazu analogen Ethersulfate nicht voll befriedigend.

Die Aufgabe der Erfindung hat demnach darin bestanden, einen Weg zu finden, Performance und Hautverträglichkeit von Monoglycerid(ether)sulfaten signifikant zu verbessern.

### Beschreibung der Erfindung

Gegenstand der Erfindung sind milde Detergensgemische, enthaltend
(a) Monoglycerid(ether)sulfate und
(b) Zuckertenside, ausgewählt aus der Gruppe, die gebildet wird von
   (b1) Saccharoseestern,
   (b2) Sorbitanestern und/oder
   (b3) Polysorbaten,
   mit der Maßgabe, daß das Gewichtsverhältnis der Komponenten (a) und (b) 75:25 bis 50:50 beträgt.

Überraschenderweise wurde gefunden, daß Abmischungen von Monoglyceridsulfaten bzw. Monoglyceridethersulfaten und den genannten Zuckertensiden in den genannten Verhältnissen zu einer in synergistischer Weise verbesserten hautkosmetischen Verträglichkeit bei gesteigertem Schaumvermögen führt.

### Monoglyceride und Monoglyceridethersulfate

Monoglyceridsulfate und Monoglyceridethersulfate stellen bekannte anionische Tenside dar, die nach den einschlägigen Methoden der präparativen organischen Chemie erhalten werden können. Üblicherweise geht man zu ihrer Herstellung von Triglyceriden aus, die gegebenenfalls nach Ethoxylierung zu den Monoglyceriden umgeestert und nachfolgend sulfatiert und neutralisiert werden. Gleichfalls ist es möglich, die Partialglyceride mit geeigneten Sulfatierungsmitteln, vorzugsweise gasförmiges Schwefeltrioxid oder Chlorsulfonsäure umzusetzen [vgl. **WO 92/09569, WO 92/09570**, Henkel]. Die neutralisierten Stoffe können - falls gewünscht - einer Ultrafiltration unterworfen werden, um den Elektrolytgehalt auf ein gewünschtes Maß zu vermindern.

Die im Sinne der Erfindung einzusetzenden **Monoglycerid(ether)sulfate** folgen der Formel (I) 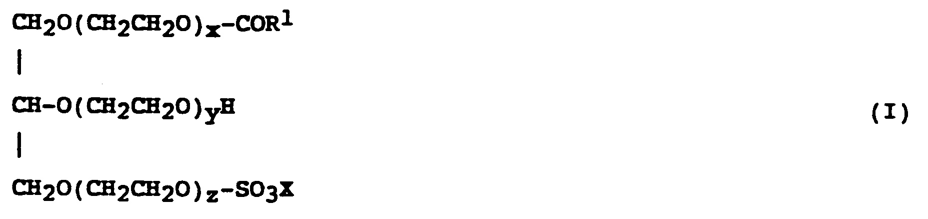 in der R¹CO für einen linearen oder verzweigten Acylrest mit 6 bis 22 Kohlenstoffatomen, x, y und z in Summe für 0 oder für Zahlen von 1 bis 30 und X für ein Alkali- oder Erdalkalimetall steht.

Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäureonoglycerid, Stearinsäuremonoglycerid, Ölsäuremonoglycerid und Talgfettsäuremonoglycerid sowie deren Ethylenoxidaddukten mit Schwefeltrioxid oder Chlorsulfonsäure in Form ihrer Natriumsalze. Vorzugsweise werden Monoglyceridsulfate der Formel (I) eingesetzt, in der R¹CO für einen linearen Acylrest mit 8 bis 18 Kohlenstoffatomen steht.

### Saccharoseester

Saccharoseester, vielfach auch als Zuckerester bezeichnet, stellen seit langer Zeit bekannte nichtionische Tenside dar. Üblicherweise erfolgt die Herstellung dieser Verbindungen über den Weg der Umesterung von Fettsäuremethylestern mit Saccharose in Gegenwart von basischen Katalysatoren wie beispielsweise Pyridin oder Piperidin in organischen Lösungsmittel [vgl. **DE-AS 2051766** (Day-Ichi; zu lösungsmittelfreien Methoden vgl. L.Guillardeau in **Tens.Surf.Det. 29, 342 (1992)**]. Je nach Auswahl der Rohstoffe und Reaktionsbedingungen entstehen dabei Mono-, Di-, Tri- und Polyester der Saccharose, wobei in der Regel nur die Mono- und Diester eine anwendungstechnische Bedeutung besitzen. Eine Übersicht zu diesem Thema ist beispielsweise von N.Desai in **Parf.Kosm. 64, 463 (1991)** erschienen, zu Struktur und Eigenschaften der Stoffe vgl. auch **Surfactants in Consumer Products, J.Falbe (ed.), Springer-Verlag, Berlin, 1987, S.101-103**.

Die Saccharoseester folgen der Formel (II), 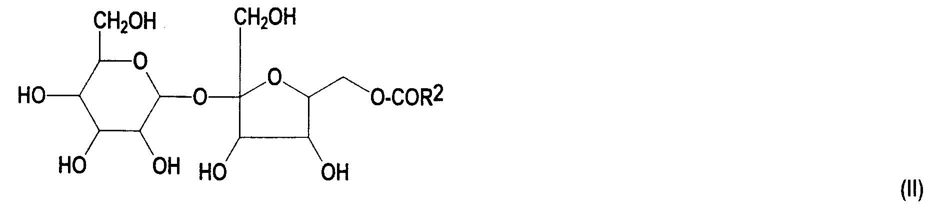 in der R²CO für einen aliphatischen Acylrest mit 6 bis 22 Kohlenstoffatomen und 0, 1, 2 oder 3 Doppelbindungen steht. Formel (II) gibt exemplarisch einen Monoester wieder. Es versteht sich, daß auch die weiteren Hydroxylgruppen, insbesondere die primären Hydroxylgruppen verestert sein können.

Typische Beispiele für geeignete Saccharoseester, vorzugsweise Saccharosemono- und/oder -diester sind Umesterungsprodukte der Saccharose mit Methylestern der Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Reduktion von Aldehyden aus der Roelen'schen Oxosynthese oder der Dimerisierung von ungesättigten Fettsäuren anfallen.

Bevorzugt sind technische Saccharosemono- und/oder -diester, deren Fettsäurekomponente sich von einem technischen C_{12/18}- bzw. C₁₂/₁₄-Kokosfettsäureschnitt ableitet.

### Sorbitanester

Sorbitanester stellen Veresterungs- bzw. Umesterungsprodukte von Sorbitol mit Fettsäuren bzw. Fettsäuremethylestern bei Temperaturen von 200 bis 250°C dar. Dabei wird in einem ersten Schritt Sorbitol zu einer Mischung von 1,4- und 3,6-Sorbitan dehydratisiert [vgl. S.Ropuszynski et al. in **Tens. Surf.Det. 27, 350 (1990)**] und das Sorbitan anschließend - abhängig von den Einsatzbedingungen - in ein Gemisch von Mono-, Di- und Triestern überführt [vgl. C.Akoh et al. in **J.Am.Oil. Chem.Soc., 66, 1581 (1989)** und **Surfactants in Consumer Products, J.Falbe (ed), Springer-Verlag, Berlin, 1987, S.101-103.**

Typische Beispiele für geeignete Sorbitanester, vorzugsweise Sorbitanmono-, Sorbitansesqui- und/oder Sorbitandiester, sind formale Veresterungsprodukte des Sorbitans mit Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Reduktion von Aldehyden aus der Roelen'schen Oxosynthese oder der Dimerisierung von ungesättigten Fettsäuren anfallen.

Bevorzugt sind technische Sorbitanmono-, -sesqui- oder -diester, deren Fettsäurekomponenten sich von einem technischen C₁₂/₁₈- bzw. C_{12/14}-Kokosfettsäureschnitt, Laurinsäure, Palmitinsäure, Stearinsäure oder Ölsäure ableiten.

### Polysorbate

Polysorbate stellen ethoxylierte Sorbitanester dar und werden üblicherweise durch nachträgliche Anlagerung von Ethylenoxid an die freien Hydroxylgruppen der Sorbitanester bzw. durch Insertion in die Carbonylesterbindungen hergestellt.

Typische Beispiele für geeignete Polysorbate, vorzugsweise ethoxylierte Sorbitanmono-, Sorbitansesqui- und/oder Sorbitandiester, sind Anlagerungsprodukte von durchschnittlich 1 bis 50, vorzugsweise 1 bis 15 Mol Ethylenoxid an jeweils 1 Mol eines formalen Veresterungsproduktes des Sorbitans mit Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Reduktion von Aldehyden aus der Roelen'schen Oxosynthese oder der Dimerisierung von ungesättigten Fettsäuren anfallen.

Bevorzugt sind technische ethoxylierte Sorbitanmono-, -sesqui- oder - diester, deren Fettsäurekomponente sich von einem technischen C_{12/18}- bzw. C_{12/14}-Kokosfettsäureschnitt, Laurinsäure, Palmitinsäure, Stearinsäure oder Ölsäure ableitet und die 10 bis 25 Mol Ethylenoxid pro Mol Ester enthalten.

### Tenside

Die erfindungsgemäßen Detergensgemische können weitere anionische, nichtionische, kationische und/oder amphotere Tenside enthalten.

Typische Beispiele für **anionische Tenside** sind Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Fettsäureamid(ether)sulfate, Mono- und Dialkylsulfosuccinate, Mono- und Dialkylsulfosuccinamate, Sulfotriglyceride, Amidseifen, Alkyloligoglucosidsulfate und Alkyl(ether)phosphate. Sofern die anionischer Tenside Polyglycoletherketten enthalten, können sie eine konventionelle, vorzugsweise jedoch eine eingeengte Homologenverteilung aufweisen.

Typische Beispiele für **nichtionische Tenside** sind Fettalkoholpolyglycolether, Alkylphenolpolyglycolether, Fettsäurepolyglycolester, Fettsäureamidpolygylcolether, Fettaminpolyglycolether, alkoxylierte Triglyceride, Mischether bzw. Mischformale, Alk(en)yloligoglykoside, Fettsäure N-alkylglucamide und Proteinhydrolysate (insbesondere pflanzliche Produkte auf Sojabasis). Sofern die nichtionischen Tenside Polyglycoletherketten enthalten, können sie eine konventionelle, vorzugsweise jedoch eine eingeengte Homologenverteilung aufweisen.

Typische Beispiele für **kationische Tenside** sind quartäre Ammoniumverbindungen und Esterquats, insbesondere quaternierte Fettsäuretrialkanolaminester-Salze.

Typische Beispiele für **amphotere bzw. zwitterionische Tenside** sind Alkylbetaine, Alkylamidobetaine, Aminopropionate, Aminoglycinate, Imidazoliniumbetaine und Sulfobetaine.

Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S.54-124** oder **J.Falbe (ed.), "Katalysatoren, Tenside und Mineralöladditive", Thieme Verlag, Stuttgart, 1978, S.123-217 verwiesen.**

Die erfindungsgemäßen Detergensgemische können die oben genannten zusätzlichen Tenside in Anteilen von 1 bis 50, vorzugsweise 5 bis 25 Gew.-% - bezogen auf den Feststoffanteil der Gemische - enthalten.

### Gewerbliche Anwendbarkeit

Die erfindungsgemäßen Detergensgemische zeichnen sich durch ein besonders vorteilhaftes Schaumvermögen und eine in synergistischer Weise verbesserte hautkosmetische Verträglichkeit aus, Eigenschaften, die bei der Entwicklung einer Vielzahl von oberflächenaktiven Mitteln von Bedeutung sind. Typische Beispiele für geeignete oberflächenaktive Mittel sind:
o **Pulverförmige Universalwaschmittel**, enthaltend 10 bis 30 Gew.-% - bezogen auf das Mittel - der erfindungsgemäßen Detergensgemische sowie übliche Hilfs- und Zusatzstoffe.
o **Flüssige Universalwaschmittel**, enthaltend 10 bis 70 Gew.-% - bezogen auf das Mittel - der erfindungsgemäßen Detergensgemische sowie übliche Hilfs- und Zusatzstoffe.
o **Flüssige Feinwaschmittel**, enthaltend 10 bis 50 Gew.-% - bezogen auf das Mittel - der erfindungsgemäßen Detergensgemische sowie übliche Hilfs- und Zusatzstoffe.
o **Avivagemittel**, enthaltend 10 bis 50 Gew.-% - bezogen auf das Mittel - der erfindungsgemäßen Detergensgemische sowie übliche Hilfs- und Zusatzstoffe.
o **Handgeschirrspülmittel**, enthaltend 10 bis 50 Gew.-% - bezogen auf das Mittel - der erfindungsgemäßen Detergensgemische sowie übliche Hilfs- und Zusatzstoffe.
o **Klarspüler**, enthaltend 10 bis 50 Gew.-% - bezogen auf das Mittel - der erfindungsgemäßen Detergensgemische sowie übliche Hilfs- und Zusatzstoffe.
o **Flüssige Reinigungs- und Desinfektionsmittel**, enthaltend 10 bis 30 Gew.-% - bezogen auf das Mittel - der erfindungsgemäßen Detergensgemische sowie übliche Hilfs- und Zusatzstoffe.
o **Stückseifen vom Kombibar-Typ**, enthaltend 1 bis 2 Gew.-% - bezogen auf das Mittel - der erfindungsgemäßen Detergensgemische sowie übliche Hilfs- und Zusatzstoffe.
o **Syndetseifen**, enthaltend 1 bis 2 Gew.-% - bezogen auf das Mittel - der erfindungsgemäßen Detergensgemische sowie übliche Hilfs- und Zusatzstoffe.
o **Haarshampoos**, enthaltend 10 bis 30 Gew.-% - bezogen auf das Mittel - der erfindungsgemäßen Detergensgemische sowie übliche Hilfs- und Zusatzstoffe.
o **Haarspülungen**, enthaltend 10 bis 30 Gew.-% - bezogen auf das Mittel - der erfindungsgemäßen Detergensgemische sowie übliche Hilfs- und Zusatzstoffe.
o **Haarfärbemittel**, enthaltend 10 bis 30 Gew.-% - bezogen auf das Mittel - der erfindungsgemäßen Detergensgemische sowie übliche Hilfs- und Zusatzstoffe.
o **Haarwellmittel,** enthaltend 10 bis 30 Gew.-% - bezogen auf das Mittel - der erfindungsgemäßen Detergensgemische sowie übliche Hilfs- und Zusatzstoffe.
o **Schaumbäder**, enthaltend 10 bis 30 Gew.-% - bezogen auf das Mittel - der erfindungsgemäßen Detergensgemische sowie übliche Hilfs- und Zusatzstoffe.
o **Textil- und Faserhilfsmittel**, enthaltend 1 bis 30 Gew.-% - bezogen auf das Mittel - der erfindungsgemäßen Detergensgemische sowie übliche Hilfs- und Zusatzstoffe.
o **Lederfettungsmittel**, enthaltend 1 bis 30 Gew.-% - bezogen auf das Mittel - der erfindungsgemäßen Detergensgemische sowie übliche Hilfs- und Zusatzstoffe.
o **Flotationshilfsmittel**, enthaltend 1 bis 30 Gew.-% - bezogen auf das Mittel - der erfindungsgemäßen Detergensgemische sowie übliche Hilfs- und Zusatzstoffe.
o **Hilfsmittel für die Feststoffentwässerung**, enthaltend 1 bis 30 Gew.-% - bezogen auf das Mittel - der erfindungsgemäßen Detergensgemische sowie übliche Hilfs- und Zusatzstoffe.

### Hilfs- und Zusatzstoffe

**Wasch-, Spül-, Reinigungs- und Avivagemittel** auf Basis der erfindungsgemäßen Detergensgemische können - neben den bereits genannten Tensiden - als weitere Hilfs- und Zusatzstoffe beispielsweise Builder, Salze, Bleichmittel, Bleichaktivatoren, optische Aufheller, Vergrauungsinhibitoren, Lösungsvermittler und Enzyme enthalten.

Übliche **Builder** sind Natriumaluminiumsilicate (Zeolithe), Phosphate, Phosphonate, Ethylendiamintetraessigsäure, Nitrilotriacetat, Citronensäure und/oder Polycarboxylate.

Als **Salze** bzw. Stellmittel kommen beispielsweise Natriumsulfat, Natriumcarbonat oder Natriumsilicat (Wasserglas) in Betracht. Als typische Einzelbeispiele für weitere Zusatzstoffe sind Natriumborat, Stärke, Saccharose, Polydextrose, TAED, Stilbenverbindungen, Methylcellulose, Toluolsulfonat, Cumolsulfonat, langkettige Seifen, Silicone, Mischether, Lipasen und Proteasen zu nennen.

**Haarshampoos, Haarlotionen** oder **Schäumbäder** können als weitere Hilfs- und Zusatzstoffe - neben den bereits genannten Tensiden - **Emulgatoren** wie etwa alkoxylierte Fettalkohole oder Sorbitanester enthalten.

Als **Überfettungsmittel** können Substanzen wie beispielsweise polyethoxylierte Lanolinderivate, Lecithinderivate und Fettsäurealkanolamide verwendet werden, wobei die letzteren gleichzeitig als Schaumstabilisatoren dienen.

Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, Polyvinylalkohol und Polyvinylpyrrolidon sowie Elektrolyte wie Kochsalz und Ammoniumchlorid.

Unter **biogenen Wirkstoffen** sind beispielsweise Pflanzenextrakte und Vitaminkomplexe zu verstehen.

Gebräuchliche **Filmbildner** sind beispielsweise Chitosan, mikrokristallines Chitosan, quaterniertes Chitosan, Polyvinylpyrrolidon, Vinylpyrrolidon-Vinyl-acetat-Copolymerisate, Polymere der Acrylsäurereihe, quaternäre Cellulose-Derivate und ähnliche Verbindungen.

Als **Konservierungsmittel** eignen sich beispielsweise Phenoxyethanol, Formaldehydlösung, Parabene, Pentadiol oder Sorbinsäure.

Als **Perlglanzmittel** kommen beispielsweise Glycoldistearinsäureester wie Ethylenglycoldistearat, aber auch Fettsäuremonoglycolester in Betracht.

Als **Farbstoffe** können die für kosmetische Zwecke geeigneten und zugelassenen Substanzen verwendet werden, wie sie beispielsweise in der Publikation **"Kosmetische Färbemittel"** der **Farbstoffkommission der Deutschen Forschungsgemeinschaft, veröffentlicht im Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen.

Ein letzter Gegenstand der Erfindung betrifft schließlich die Verwendung der erfindungsgemäßen Detergensgemische zur Herstellung von flüssigen oder festen oberflächenaktiven Mitteln, in denen sie in Mengen von 1 bis 99 und vorzugsweise 10 bis 90 Gew.-% - bezogen auf den Feststoffgehalt der Mittel - enthalten sein können.

### Beispiele

### I. Eingesetzte Tenside

A1) C₁₂/₁₈-Kokosmonoglyceridsulfat-Natriumsalz
B1) Saccharose-mono/di-laurat
B2) Sorbitanmonolaurat
   DEHYMULS^{(R)} SML, Henkel KGaA, Düsseldorf/FRG
B3) Polysorbat (Sorbitanmonolaurat-20 EO) EUMULGIN^{(R)} SML-20. Henkel KGaA, Düsseldorf/FRG

### II. Anwendungstechnische Ergebnisse

Das Schaumvermögen wurde nach der DIN-Methode 53 902, Teil 2 (Ross-Miles-Test) durchgeführt. Eingesetzt wurden 1 Gew.-%ige Tensidlösungen in Wasser von 16°d; die Temperatur betrug 20°C. Bestimmt wurden Basisschaum und Schaumvolumen nach 5 min.

Die Bestimmung des Reizpotentials erfolgte gemaß der OECD-Methode No.404 und der EEC Directive 84/449 EEC, Pt.B.4. Die angegebenen Reizsummenscores wurden aus den nach 24, 48 und 72 Stunden erhaltenen Reizscores gebildet. Dabei wurde der im Vergleichsversuch V1 ermittelte Reizsummenscore für ein 100 %iges C_{12/18}-Kokosfettsäuremonoglyceridsulfat-Natriumsalz zu 100 % gesetzt und die in den übrigen Versuchen erhaltenen Reizsummenscores zu diesem ins Verhältnis gesetzt.

Die Ergebnisse sind in Tabelle 1 zusammengefaßt (Prozentangaben als Gew.-%).

**Tabelle 1**

| Schaumvermögen und Reizpotential | | | | | | |
|---|---|---|---|---|---|---|
| **Bsp.** | **[Al]** | **B** | **[B]** | **Schaumhöhe [ml]** | | **Reizsummen score %-rel** |
| | | | | **sofort** | **nach 5 min** | |
| 1 | 50 | B1 | 50 | 510 | 390 | 60 |
| 2 | 70 | B1 | 30 | 540 | 400 | 55 |
| 3 | 70 | B2 | 30 | 530 | 390 | 55 |
| 4 | 70 | B3 | 30 | 550 | 410 | 55 |
| V1 | 100 | - | - | 500 | 300 | 100 |
| V2 | 0 | B1 | 100 | 200 | 100 | 45 |
| V3 | 0 | B2 | 100 | 200 | 100 | 48 |
| V4 | 0 | B3 | 100 | 250 | 110 | 43 |

## Patentansprüche

1. Milde Detergensgemische, enthaltend
(a) Monoglycerid(ether)sulfate und
(b) Zuckertenside, ausgewählt aus der Gruppe, die gebildet wird von
(b1) Saccharoseestern,
(b2) Sorbitanestern und/oder
(b3) Polysorbaten,
mit der Maßgabe, daß das Gewichtsverhältnis der Komponenten (a) und (b) 75 : 25 bis 50:50 beträgt.

2. Detergensgemische nach Anspruch 1, **dadurch gekennzeichnet**, daß sie Monoglycerid(ether)sulfate der Formel **(I)** enthalten, 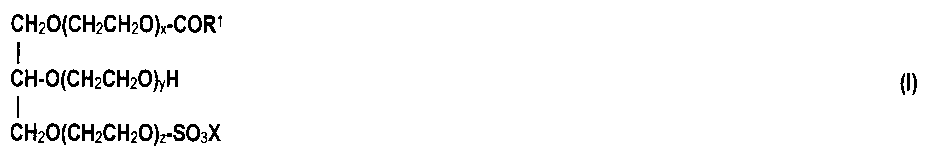 in der R¹CO für einen linearen oder verzweigten Acylrest mit 6 bis 22 Kohlenstoffatomen, x, y und z in Summe für 0 oder für Zahlen von 1 bis 30 und X für ein Alkali- oder Erdalkalimetall steht.

3. Detergensgemische nach den Ansprüchen 1 und 2, **dadurch gekennzeichnet**, daß sie Zuckerester der Formel **(II)** enthalten, 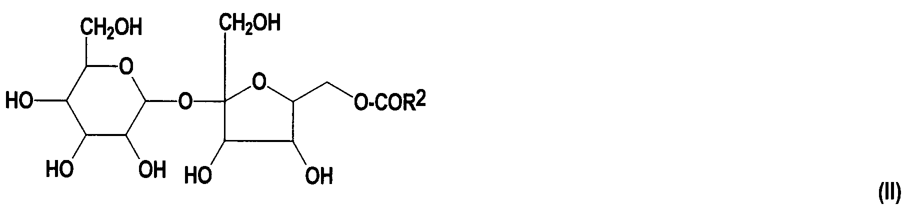 in der R²CO für einen aliphatischen Acylrest mit 6 bis 22 Kohlenstoffatomen und 0, 1, 2 oder 3 Doppelbindungen steht.

4. Verwendung der Detergensgemische nach Anspruch 1 zur Herstellung von oberflächenaktiven Mitteln.

## Claims

1. Mild detergent mixtures containing
**(a)** monoglyceride (ether) sulfates and
**(b)** sugar surfactants selected from the group consisting of
(b1) sucrose esters,
(b2) sorbitan esters and/or
(b3) polysorbates,
with the proviso that the ratio by weight of component (a) to component (b) is 75:25 to 50:50.

2. Detergent mixtures as claimed in claim 1, characterized in that they contain monoglyceride (ether) sulfates corresponding to formula (I): 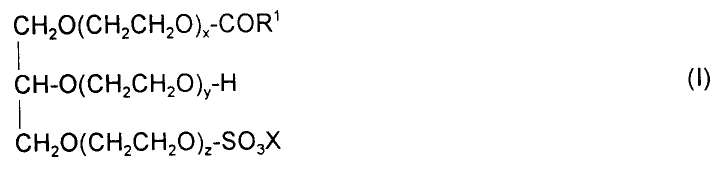 in which R¹CO is a linear or branched acyl group containing 6 to 22 carbon atoms, x, y and z together stand for 0 or for a number of 1 to 30 and X is an alkali metal or alkaline earth metal.

3. Detergent mixtures as claimed in claims 1 and 2, characterized in that they contain sugar esters corresponding to formula (II): 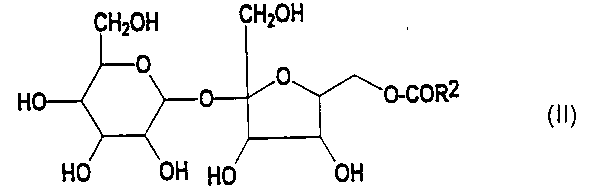 in which R²CO is an aliphatic acyl group containing 6 to 22 carbon atoms and 0, 1, 2 or 3 double bonds.

4. The use of the detergent mixtures claimed in claim 1 for the production of surface-active formulations.

## Revendications

1. Mélanges de détergents doux, renfermant
(a) des (éther)sulfates de monoglycérides et
(b) des tensioactifs sacchariques sélectionnés parmi le groupe, qui est formé des
(b1) esters de saccharose,
(b2) esters de sorbitane et/ou
(b3) polysorbates,
à condition que le rapport pondéral des composants (a) et (b) atteigne 75:25 à 50:50.

2. Mélanges de détergents selon la revendication 1, **caractérisés en ce qu**'ils renferment des (éther)sulfates de monoglycérides de la formule (I) 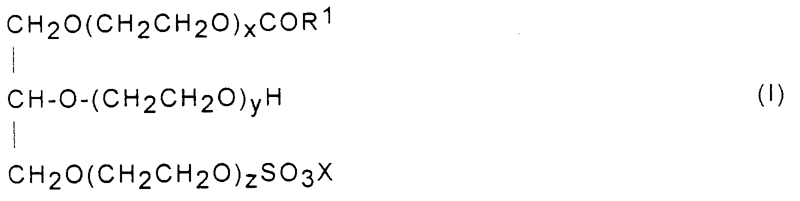 dans laquelle R¹CO représente un radical acyle linéaire ou ramifié, comportant 6 à 22 atomes de carbone, la somme de x, y et z est égale à O ou à un nombre de 1 à 30 et X est un métal alcalin ou alcalino-terreux.

3. Mélanges de détergents selon les revendications 1 et 2, **caractérisés en ce qu**'ils renferment des esters sacchariques de la formule (II) 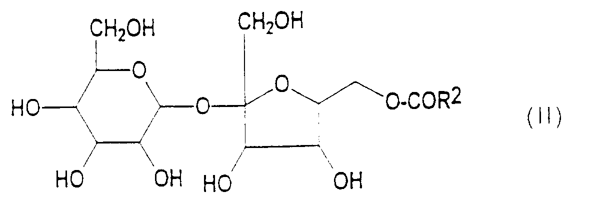 dans laquelle R²CO représente un radical acyle aliphatique comportant 6 à 22 atomes de carbone et 0, 1, 2 ou 3 doubles liaisons.

4. Utilisation des mélanges de détergents selon la revendication 1 pour la production de produits tensioactifs.
